(19) Europäisches Patentamt European Patent Office Office européen des brevets

(11) **EP 3 892 256 B1**

(12) **EUROPEAN PATENT SPECIFICATION**

(45) Date of publication and mention of the grant of the patent:
**20.12.2023 Bulletin 2023/51**

(21) Application number: **19893280.8**

(22) Date of filing: **03.10.2019**

(51) International Patent Classification (IPC):
*A61K 8/39* (2006.01)     *A61Q 1/14* (2006.01)
*A61K 8/92* (2006.01)     *A61K 8/86* (2006.01)
*A61K 8/37* (2006.01)

(52) Cooperative Patent Classification (CPC):
**A61K 8/37; A61K 8/39; A61Q 1/14;**
A61K 2800/262; A61K 2800/5922; A61K 2800/596

(86) International application number:
**PCT/JP2019/039114**

(87) International publication number:
**WO 2020/116011 (11.06.2020 Gazette 2020/24)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2018 JP 2018229932**

(43) Date of publication of application:
**13.10.2021 Bulletin 2021/41**

(73) Proprietor: **Taiyo Kagaku Co., Ltd.**
**Mie 512-1111 (JP)**

(72) Inventors:
• **MATSUMOTO, Yoshiyuki**
**Yokkaichi-shi,**
**Mie (JP)**
• **ITO, Satoko**
**Yatomi-shi,**
**Aichi (JP)**
• **HIGUCHI, Tomonori**
**Yokkaichi-shi,**
**Mie (JP)**

(74) Representative: **Vossius & Partner**
**Patentanwälte Rechtsanwälte mbB**
**Siebertstraße 3**
**81675 München (DE)**

(56) References cited:
**EP-A1- 2 932 957**     **JP-A- 2004 035 420**
**JP-A- 2005 162 691**     **JP-A- 2006 347 900**
**JP-A- 2014 083 514**     **JP-A- 2014 122 198**
**JP-A- 2014 210 744**

• **DATABASE WPI Week 200429 Thomson Scientific, London, GB; AN 2004-308463 XP002808185, & JP 2004 035420 A (SAKAMOTO YAKUHIN KOGYO KK) 5 February 2004 (2004-02-05)**
• **DATABASE WPI Week 201780 Thomson Scientific, London, GB; AN 2017-80198W XP002808184, & JP 2017 206450 A (FANCL CORP) 24 November 2017 (2017-11-24)**

**Description**

TECHNICAL FIELD

The present invention relates to a cosmetic composition and cosmetics containing the composition.

BACKGROUND ART

In the fields of cosmetics, cleansing cosmetics for makeup removal are classified by their into creams, milky lotions, gels, and liquids, and classified by their types to emulsion types, oil types, and aqueous types, which have been utilized according to their usefulness. With the changes in the life-styles of the recent years, consumers have desired as the dosage forms for removing makeups conveniently without taking much time, cleansing lotions that are contained in absorbent cottons (cotton wools) or previously soaked in nonwoven fabrics arc used for wipe off over makeups. Since the cleansing actions are carried out with lotions or the like without washing faces after the use of this dosage form, the refreshed feeling with reduced stickiness has been desired.

In aqueous cleansing cosmetics such as cleansing lotions, since the makeup products are oil-soluble, the cleansing power is less advantageous in many cases, as compared to those of the oil type or emulsion type cleansing cosmetics. Accordingly, the techniques of improving cleansing power of the aqueous cleansing cosmetics have been desired. For example, in transparent cleansing cosmetics of an aqueous type, as the technique of providing cosmetics which are more likely to be compatible with makeup stains to quickly rise up the stains, and have excellent cleansing power, the present applicant has proposed a cosmetic composition characterized in that the cosmetic composition comprises a polyglycerol medium-chain fatty acid ester made from an esterification reaction of 1.0 mol of a polyglycerol having an average degree of polymerization of from 3 to 100 and 1.2 to 3.5 mol of a medium-chain fatty acid having from 6 to 12 carbon atoms (Patent Publication 1).

However, the compatibility with makeups or the cleansing power of the prior art is not as satisfactory, as compared to those of oil type cleansing cosmetics, and further improvements have been in demand. Also, if the surfactant amount is increased in order to improve cleansing power, the stickiness distinctively owned by the surfactant after use is likely to be felt, thereby making it difficult to also satisfy the refreshed feeling.

PRIOR ART REFERENCES

PATENT PUBLICATIONS

Patent Publication 1: Japanese Patent Laid-Open No. 2014-210744

SUMMARY OF THE INVENTION

PROBLEMS TO BE SOLVED BY THE INVENTION

Although the cosmetic composition of Patent Publication 1 had excellent cleansing power or the like, further improvements are desired. As a result of intensive studies, the present inventors have newly found that excellent cleansing power and compatibility with makeups are exhibited, and the refreshed feeling with reduced stickiness is obtained by combining particular two kinds of surfactants and further blending an oil agent therewith.

An object of the present invention is to provide a cosmetic composition having excellent cleansing power and compatibility with makeups, and low stickiness, and cosmetic containing the composition.

MEANS TO SOLVE THE PROBLEMS

The present invention relates to the following [1] to [2]:

[1] A cosmetic composition containing the following components (A) to (D):

Component (A): a polyglycerol fatty acid ester made from a fatty acid having 8 carbon atoms and a polyglycerol having a degree of polymerization of glycerol of from 3 to 14;
Component (B): a polyglycerol fatty acid ester made from a fatty acid having from 10 to 12 carbon atoms and a polyglycerol having a degree of polymerization of glycerol of from 3 to 14;
Component (C): a liquid oil; and
Component (D): water,

wherein a total amount of Component (A) and Component (B) is from 1 to 7.5% by mass, and wherein the mass ratio of Component (C) to a total amount of Components (A) and (B) ((C)/(A)+(B)) is from 1/15 to 1, and wherein Component (A) and Component (B) have a mixed HLB of from 11.5 to 14.0; and wherein the content of said Component (D) is 88.5% by mass or more.

[2] Cosmetics containing a cosmetic composition as defined in [1].

EFFECTS OF THE INVENTION

The present invention provides a cosmetic composition having excellent cleansing power and compatibility with makeups, and low stickiness, and cosmetic containing the composition.

BRIEF DESCRIPTION OF THE DRAWINGS

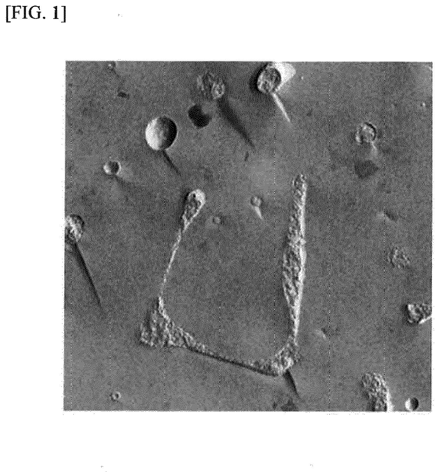

[FIG. 1] FIG. 1 shows the observational results of the cosmetic composition of Example 31 with a transmission electron microscope.

MODES FOR CARRYING OUT THE INVENTION

Although the reasons why the cleansing effects are exhibited in the present invention are not fully elucidated, it is assumed that the formation of an association body which is more likely to penetrate makeups by combining two kinds of surfactants having high abilities of reducing surface tensions contributes to the cleansing power and the compatibility with makeups.

The cosmetic composition of the present invention contains two kinds of specified polyglycerol fatty acid esters as Components (A) and (B), a liquid oil as Component (C), and water as Component (D).

Component (A) is an ester made from a fatty acid having 8 carbon atoms and a polyglycerol having a degree of polymerization of glycerol of from 3 to 14, and preferably from 6 to 14.

Component (B) is an ester made from a fatty acid having from 10 to 12 carbon atoms and a polyglycerol having a degree of polymerization of glycerol of from 3 to 14, and preferably from 3 to 10.

The stability when blended with an oil agent and the excellent cleansing power and compatibility with makeups can be both satisfied by a combined use of Components (A) and (B).

The method of calculating an average degree of polymerization of the polyglycerol in Components (A) and (B) is determined by a hydroxyl value in accordance with the following equation. Also, the method of determining the number of moles of the polyglycerol is obtained by calculating a molecular weight from an average degree of polymerization, and calculating the number of moles.

$$OHV = 56110 \, (n + 2) \, / \, (74n + 18)$$

OHV: hydroxyl value of polyglycerol
n: average degree of polymerization of polyglycerol

The polyglycerol fatty acid ester is easily obtained by an esterification of a polyglycerol and a fatty acid by a conventional method, or an addition polymerization of a fatty acid and a glycidol. The esterification of a polyglycerol and a fatty acid can be carried out by, but not particularly limited to, for example, heating a polyglycerol and a fatty acid at a temperature range of preferably from 100° to 300°C, and more preferably from 120° to 260°C, in the presence of an acid catalyst (phosphoric acid, p-toluenesulfonic acid, or the like) or an alkali catalyst (sodium hydroxide, or the like), or in the absence of a catalyst, while removing water. In addition, the reaction may be carried out in the presence of an inert gas. The ester obtained as described above may be purified in accordance with its purposes. As the purification, in addition to the distillation technique such as distillation under a reduced pressure, a molecular distillation, or a steam distillation, an extraction with an organic solvent, fractionations, or chromatography separations with column packed with synthetic adsorbents or gel filtration agents can be utilized. Here, an intended polyglycerol fatty acid ester may be obtained by carrying out a transesterification with a polyglycerol using a fatty acid ester in place of a fatty acid.

The mass ratio of Component (A) to Component (B) in the cosmetic composition of the present invention is preferably from 9:1 to 1:9, from the viewpoint of cleansing power and stability. In addition, a total amount of Component (A) and Component (B) in the cosmetic composition of the present invention is from 1 to 7.5% by mass, preferably from 2 to 7.5% by mass, and more preferably from 3 to 5% by mass, from the viewpoint of the compatibility with makeups and the stickiness. In addition, a mixed HLB of Component (A) and Component (B) is from 11.5 to 14.0, and preferably from 13.0 to 14.0, from the viewpoint of cleansing power and the compatibility with makeups.

The HLB as used herein refers to a value measured by a Griffin calculation method given below, and a mixed HLB of Component (A) and Component (B) is calculated utilizing the additive properties thereof. Specifically, the mixed HLB as used herein is a weighted average of Component (A) and Component (B).

$$HLB = 20\ (1 - S/A)$$

wherein S: saponification value of a polyglycerol fatty acid ester, and

A: a neutral value of a raw material fatty acid.

Component (C) is a liquid oil. The liquid oil includes hydrocarbon oils, ester oils, acyl glycerols, ether oils, plant or animal oils, silicone oils, and the like, and the ester oils are preferred, from the viewpoint of stickiness.

The hydrocarbon oil includes hydrogenated polyisobutenes, liquid parrafins, light liquid parrafins, squalane, undecane, tridecane, and the like.

The ester oil includes isononyl isononanoate, isostearyl isostearate, cetyl 2-ethylhexanoate, 2-ethylhexyl palmitate, octyldodecyl myristate, isopropyl myristate, propylheptyl caprylate, caprylyl caprylate/caprate, coco caprylate/caprate, dicaprylyl carbonate, and the like.

The acyl glycerol includes glyceryl tri(caprylate/caprate), glyceryl tri-2-ethylhexanoate, glyceryl triisostearate, glyceryl diisostearate, and the like.

The plant or animal oil includes avocado oil, almond oil, olive oil, wheat germ oil, rice germ oil, rice bran oil, safflower oil, soybean oil, Indian corn oil, rapeseed oil, palm oil, palm kernel oil, castor oil, sunflower oil, jojoba oil, macadamia nut oil, coconut oil, lanolin, and the like.

The silicone oil includes dimethicone, phenyl trimethicone, cyclomethicone, cyclopentasiloxane, and the like.

The content of Component (C) in the cosmetic composition of the present invention is preferably 1% by mass or more, from the viewpoint of skin texture after use, and the content is preferably 3% by mass or less, from the viewpoint of the feel distinctively owned by the oily component. The content of Component (C) when two or more kinds are used refers to a total amount thereof.

The mass ratio of Component (C) to a total amount of Components (A) and (B) in the cosmetic composition of the present invention, i.e., (C) /(A) + (B), is from 1/15 to 1, and preferably from 1/15 to 3/5, from the viewpoint of obtaining a transparent external appearance.

Component (D) is water. The content of Component (D) in the cosmetic composition of the present invention is 88.5% by mass or more.

The cosmetic composition of the present invention can further contain a hydrophilic nonionic surfactant as Component (E). Since the cosmetic composition contains Component (E), the cosmetic composition has excellent high-temperature stability.

Component (E) includes polyglycerol alkyl ethers, polyglycerol fatty acid esters, alkyl polyglucosides, polyoxyethylene alkyl ethers, polyoxyethylene sorbitan monoalkylates, and the like, and the polyglycerol alkyl ethers are preferred, from the viewpoint of high-temperature stability.

The polyglycerol alkyl ether is preferably an ether with an alkyl group having 12 carbon atoms, which includes polyglycerol lauryl ethers, and the like. Among them, monoethers with a polyglycerol having a degree of polymerization of from 3 to 6 are even more preferred.

The content of Component (E) in the cosmetic composition of the present invention is preferably 0.01% by mass or more, and more preferably 0.1% by mass or more, from the viewpoint of high-temperature stability, and the content is preferably 1.0% by mass or less, and more preferably 0.5% by mass or less, from the same viewpoint.

The cosmetic composition of the present invention can be blended in proper amounts with components usually used in cosmetics in accordance with their applications and purposes, within the range that would not impair the effects of the present invention. The components include, but not limited to, for example, surfactants other than Components (A) and (B), oil agents other than Component (C), aqueous gelating agents, oily gelating agents, ultraviolet absorbents, powders, antioxidants, preservatives, perfumes, colorants, chelating agents, refreshing agents, thickening agents, plant extracts, vitamins, neutralizing agents, moisturizing agents, anti-inflammatory agents, pH adjusting agents, amino acids, and the like.

The method for preparing a cosmetic composition of the present invention is not particularly limited. For example, the cosmetic composition can be prepared by a method including, for example, dissolving all the components at 70°C, and cooling the solution to room temperature.

Since the cosmetic composition of the present invention preferably has a transparent external appearance, excellent cleansing power, and low stickiness, the cosmetic composition can be suitably used as aqueous cleansing cosmetics, and in particular suitably used as wipe-off type cleansing cosmetics. In other words, the present invention also provides cosmetics containing a cosmetic composition of the present invention.

The cosmetics of the present invention can be prepared by blending various components usually used in cosmetics to a cosmetic composition in accordance with the applications and purposes thereof, to give cosmetics. The components include, but not limited to, for example, surfactants other than Components (A) and (B), oil agents other than Component (C), polyhydric alcohols, aqueous gelating agents, oily gelating agents, ultraviolet absorbents, powders, antioxidants, preservatives, perfumes, colorants, chelating agents, refreshing agents, thickening agents, plant extracts, vitamins, neutralizing agents, moisturizing agents, anti-inflammatory agents, pH adjusting agents, amino acids, and the like.

The content proportion of the cosmetic composition of the present invention in the cosmetics is, but not limited to, preferably from 10 to 100% by mass, more preferably from 20 to 100% by mass, and even more preferably from 50 to 100% by mass, from the viewpoint of cleansing power.

EXAMPLES

The examples of the present invention will be described more specifically hereinbelow, without intending to limit the present invention to these examples. Here, "%" means "% by mass" unless specified otherwise.

Preparation of Cosmetic Compositions

Examples 1 to 30 and Comparative Examples 1 to 10

Components shown in Tables 1 to 4 in a composition listed in the tables were heated to dissolve at 70°C, and the mixed solution was then cooled to room temperature while stirring, to produce 100 g of each of cosmetic compositions. Here, the oils (Component (C)) used in Tables 1, 2, and 4 were all isononyl isononanoate.

The details of the components used in Tables 1 to 4 are shown below. Here, each of the polyglycerol fatty acid esters is synthesized by a known means.

Isononyl isononanoate (manufactured by The Nissin OilliO Group, Ltd.)
Hydrogenated polyisobutene (manufactured by NOF Corporation)
Liquid paraffin (manufactured by MORESCO Corporation)
Triethylhexanoin (manufactured by Taiyo Kagaku Co., Ltd.)
Olive oil (manufactured by CRODA Japan)
Cyclic siloxane (manufactured by Shin-Etsu Silicone)
Polyglycerol-4 lauryl ether (manufactured by Taiyo Kagaku Co., Ltd.)
Polysolvate 20 (manufactured by Kao Corporation)
Laureth-15 (manufactured by Nihon Emulsion Co., Ltd.)
Lauryl glucoside (manufactured by Kao Corporation)

With respect to each of the cosmetic compositions obtained in Examples and Comparative Examples, the state of external appearance, the cleansing power, the compatibility with makeups, the feel when drying, and the feel after drying were assessed in accordance with the following criteria. The results are shown in Tables 1 to 4.

< Assessment of State of External Appearance >

A quartz cell was charged with a cosmetic composition, and the transmittance, %, at a wavelength of 660 nm was measured with a spectrophotometer (Hitachi High-Tech Science Corporation, U-3900), with purified water as a control sample.

(Assessment Criteria)

◎: transmittance being 90% or more;
○: transmittance being 80% or more and less than 90%;
△: transmittance being 60% or more and less than 80%; and
×: transmittance being less than 60%.

< Assessment of Compatibility with Makeups >

An oil-soluble pigment (Sudan III) was added to a cosmetic composition placed in a vial, and the external appearance of the mixture after one day was visually observed. The more excellent the staining of the oily pigment, the lower the surface tension, which was assessed to have excellent compatibility with makeups.

(Assessment Criteria)

4:    the entirety of the system being stained with an oily pigment;

3:    a half of the system being stained with an oily pigment;

2:    a part of the system being stained with an oily pigment; and

1:    the system not being stained with an oily pigment at all.

< Assessment of Cleansing Power >

Mascara (water-proof type) was applied to a bio-skin, and allowed to stand for two hours. The mascara was wiped three times with a cosmetic composition previously soaked in cotton absorbents (cotton wools). The visual observation results of the extent of mascara removal were assessed in accordance with the assessment criteria, and defined as cleansing power at the beginning of wipe off.

(Assessment Criteria)

4:    well removed;
3:    removed;
2:    not very well removed; and
1:    not removed at all.

< Assessments of Stickiness >

As the assessments of stickiness, the feel when drying and the feel after drying were each assessed.

Feel When Drying

Five specialized panelists were asked to apply a cosmetic composition previously soaked in the cotton absorbents (cotton wools) over the entire face, and assess the skin feel when drying in the following four stages. Here, the assessments were an average of the assessments of the five panelists, and those of 2.5 or higher can be assessed as low stickiness.

(Assessment Criteria)

4:    felt smoothness;
3:    slightly felt stickiness;
2:    felt stickiness; and
1:    considerably felt stickiness.

Feel After Drying

Five specialized panelists were asked to apply a cosmetic composition soaked in the cotton absorbents (cotton wools) over the entire face, and assess the skin feel after drying in the following four stages. Here, the assessments were an average of the assessments of the five panelists, and those of 2.5 or higher can be assessed as low stickiness.

(Assessment Criteria)

4: felt smoothness;
3: slightly felt stickiness;
2: felt stickiness; and
1: considerably felt stickiness.

< High-Temperature Stability >

A cosmetic composition placed in a vial was gradually heated in a water bath to raise its temperature, and a temperature at which the cosmetic composition turned into white turbid was measured. The higher the temperature at which the cosmetic composition turned into white turbid, the cosmetic composition was assessed as having excellent high-temperature stability.

< Skin Irritability >

A cosmetic composition was soaked in cotton absorbents (cotton wools), and the feel when the cotton absorbents were pressed against the tail of the eyes for one minute was assessed in accordance with the following criteria. Here, the assessments were an average of the assessments by eight panelists, and those of 3.0 or more can be assessed as low irritability.

(Assessment Criteria)

4: no irritable feel, agreeable feel
3: somewhat a disagreeable feel such as a cool refreshed feel, a warm feel, or stiff feel being found
2: a feeling itchiness or feel of sensory irritations to an extent that is barely discernible; and
1: distinct sensory irritations (tingles, pungent feel, itchiness).

[Table 1]

Table 1

| | Compound Name | Blending Amount of Components A, B, % by mass | Kind of Fatty Acid | Average Degree of Polymerization of Polyglycerol | HLB | Mixed HLB | Blending Amount of Oil, % by mass | Blending Amount of Water, % by mass | C/(A+B) | State of External Appearance | Compatibility with Makeups | Cleansing Power | Anti-Irritating Property | Feel When Drying | Feel After Drying |
|---|---|---|---|---|---|---|---|---|---|---|---|---|---|---|---|
| Comp. Ex. 1 | Tetradecaglycerol caprylate | 4 | C8 | 14 | 16.1 | 15.0 | 1 | 94 | 1/5 | × | 1 | 1 | not assessable | not assessable | not assessable |
| | Hexaglycerol dicaprate | 1 | C10 | 6 | 10.7 | | | | | | | | | | |
| Comp. Ex. 2 | Hexaglycerol caprylate | 5 | C8 | 6 | 14.5 | 14.5 | 1 | 94 | 1/5 | △ | 2 | 2 | 2.6 | 2.8 | 2.6 |
| Comp. Ex. 3 | Hexaglycerol caprylate | 3 | C8 | 6 | 14.5 | 14.1 | 1 | 94 | 1/5 | △ | 2 | 2 | 2.5 | 2.8 | 2.4 |
| | Hexaglycerol sesquicaprate | 2 | C10 | 6 | 13.5 | | | | | | | | | | |
| Ex. 1 | Tetradecaglycerol caprylate | 3 | C8 | 14 | 16.1 | 13.9 | 1 | 94 | 1/5 | ◎ | 3 | 3 | 3.2 | 2.6 | 2.8 |
| | Hexaglycerol dicaprate | 2 | C10 | 6 | 10.7 | | | | | | | | | | |
| Ex. 2 | Hexaglycerol caprylate | 4.5 | C8 | 6 | 14.5 | 13.9 | 1 | 94 | 1/5 | ○ | 3 | 3 | 3.1 | 2.6 | 2.6 |
| | Hexaglycerol trilaurate | 0.5 | C12 | 6 | 8.9 | | | | | | | | | | |
| Ex. 3 | Hexaglycerol caprylate | 4 | C8 | 6 | 14.5 | 13.7 | 1 | 94 | 115 | ◎ | 4 | 4 | 3.3 | 3.4 | 3.6 |
| | Hexaglycerol dicaprate | 1 | C10 | 6 | 10.7 | | | | | | | | | | |

| | Compound Name | Blending Amount of Components A, B, % by mass | Kind of Fatty Acid | Average Degree of Polymerization of Polyglycerol | HLB | Mixed HLB | Blending Amount of Oil, % by mass | Blending Amount of Water, % by mass | C/(A+B) | State of External Appearance | Compatibility with Makeups | Cleansing Power | Anti-Irritating Property | Feel When Drying | Feel After Drying |
|---|---|---|---|---|---|---|---|---|---|---|---|---|---|---|---|
| Ex. 4 | Hexaglycerol caprylate | 0.5 | C8 | 6 | 14.5 | 13.6 | 1 | 94 | 1/5 | ◎ | 4 | 4 | 3.3 | 3.4 | 3.4 |
| | Hexaglycerol sesquicaprate | 4.5 | C10 | 6 | 13.5 | | | | | | | | | | |
| Comp. Ex. 4 | Hexaglycerol sesquicaprate | 5 | C10 | 6 | 13.5 | 13.5 | 1 | 94 | 1/5 | △ | 2 | 3 | 3.0 | 2.8 | 3.2 |
| Ex. 5 | Hexaglycerol caprylate | 3 | C8 | 6 | 14.5 | 13.4 | 1 | 94 | 1/5 | ◎ | 4 | 4 | 3.4 | 3.0 | 3.2 |
| | Pentaglvcerol tricaprate | 2 | C10 | 5 | 11.7 | | | | | | | | | | |
| Ex. 6 | Pentaglycerol dicaprylate | 3 | C8 | 5 | 14.8 | 13.2 | 1 | 94 | 1/5 | ◎ | 4 | 4 | 3.3 | 2.8 | 3.0 |
| | Hexaglycerol dicaprate | 2 | C10 | 6 | 10.7 | | | | | | | | | | |
| Ex. 7 | Hexaglycerol caprylate | 3 | C8 | 6 | 14.5 | 13.1 | 1 | 94 | 1/5 | ◎ | 4 | 3 | 3.4 | 2.6 | 2.8 |
| | Tetradecaglycerol tetracaprate | 2 | C10 | 14 | 11.1 | | | | | | | | | | |
| Ex. 8 | Hexaglycerol caprylate | 3 | C8 | 6 | 14.5 | 13.0 | 1 | 94 | 1/5 | ◎ | 4 | 3 | 3.3 | 3.6 | 3.4 |
| | Hexaglycerol dicaprate | 2 | C10 | 6 | 10.7 | | | | | | | | | | |
| Ex. 9 | Triglycerol caprylate | 3 | C8 | 3 | 11.6 | 12.4 | 1 | 94 | 1/5 | ◎ | 3 | 3 | 3.0 | 3.0 | 3.4 |
| | Hexaglycerol sesquicaprate | 2 | C10 | 6 | 13.5 | | | | | | | | | | |

(continued)

| | Compound Name | Blending Amount of Components A, B, % by mass | Kind of Fatty Acid | Average Degree of Polymerization of Polyglycerol | HLB | Mixed HLB | Blending Amount of Oil, % by mass | Blending Amount of Water, % by mass | C/(A+B) | State of External Appearance | Compatibility with Makeups | Cleansing Power | Anti-Irritating Property | Feel When Drying | Feel After Drying |
|---|---|---|---|---|---|---|---|---|---|---|---|---|---|---|---|
| Ex. 10 | Hexaglycerol caprylate | 2 | C8 | 6 | 14.5 | 12.2 | 1 | 94 | 1/5 | ◎ | 3 | 3 | 3.2 | 3.2 | 3.6 |
| | Hexaglycerol dicaprate | 3 | C10 | 6 | 10.7 | | | | | | | | | | |
| Ex. 11 | Hexaglycerol dicaprylate | 3 | C8 | 6 | 12.3 | 11.7 | 1 | 94 | 1/5 | ◎ | 3 | 3 | 3.1 | 3.2 | 3.2 |
| | Hexaglycerol dicaprate | 2 | C10 | 6 | 10.7 | | | | | | | | | | |
| Comp. Ex. 5 | Hexaglycerol dicaprylate | 2 | C8 | 6 | 12.3 | 11.3 | 1 | 94 | 1/5 | × | 1 | 3 | not assessable | not assessable | not assessable |
| | Hexaglycerol dicaprate | 3 | C10 | 6 | 10.7 | | | | | | | | | | |

[Table 2]

EP 3 892 256 B1

Table 2

| | Compound Name | Blending Amount of Components A, B, % by mass | Kind of Fatty Acid | Average Degree of Polymerization of Polyglycerol | HLB | Mixed HLB | Blending Amount of Oil, % by mass | Blending Amount of Water, % by mass | C/(A+B) | State of External Appearance | Compatibility with Makeups | Cleansing Power | Feel When Drying | Feel After Drying |
|---|---|---|---|---|---|---|---|---|---|---|---|---|---|---|
| Ex. 12 | Hexaglycerol caprylate | 1.2 | C8 | 6 | 14.5 | 13.0 | 0.5 | 97.5 | 1/4 | ◎ | 3 | 3 | 2.6 | 2.8 |
| | Hexaglycerol dicaprate | 0.8 | C10 | 6 | 10.7 | | | | | | | | | |
| Ex. 13 | Hexaglycerol caprylate | 1.8 | C8 | 6 | 14.5 | 13.0 | 1 | 96 | 1/3 | ◎ | 4 | 4 | 3.4 | 3.4 |
| | Hexaglycerol dicaprate | 1.2 | C10 | 6 | 10.7 | | | | | | | | | |
| Comp. Ex. 6 | Hexaglycerol caprylate | 3 | C8 | 6 | 14.5 | 13.0 | 0 | 95 | 0 | ○ | 1 | 2 | 1.2 | 1.6 |
| | Hexaglycerol dicaprate | 2 | C10 | 6 | 10.7 | | | | | | | | | |
| Comp. Ex. 7 | Hexaglycerol caprylate | 3 | C8 | 6 | 14.5 | 13.0 | 0.25 | 94.75 | 1/20 | ○ | 2 | 2 | 1.8 | 2.4 |
| | Hexaglycerol dicaprate | 2 | C10 | 6 | 10.7 | | | | | | | | | |
| Ex. 14 | Hexaglycerol caprylate | 3 | C8 | 6 | 14.5 | 13.0 | 0.5 | 94.5 | 1/10 | ○ | 3 | 4 | 3.0 | 3.2 |
| | Hexaglycerol dicaprate | 2 | C10 | 6 | 10.7 | | | | | | | | | |
| Ex. 15 | Hexaglycerol caprylate | 3 | C8 | 6 | 14.5 | 13.0 | 2 | 93 | 2/5 | ○ | 4 | 4 | 3.6 | 3.4 |
| | Hexaglycerol dicaprate | 2 | C10 | 6 | 10.7 | | | | | | | | | |

12

| | Compound Name | Blending Amount of Components A, B, % by mass | Kind of Fatty Acid | Average Degree of Polymerization of Polyglycerol | HLB | Mixed HLB | Blending Amount of Oil, % by mass | Blending Amount of Water, % by mass | C/(A+B) | State of External Appearance | Compatibility with Makeups | Cleansing Power | Feel When Drying | Feel After Drying |
|---|---|---|---|---|---|---|---|---|---|---|---|---|---|---|
| Ex. 16 | Hexaglycerol caprylate | 3 | C8 | 6 | 14.5 | 13.0 | 3 | 92 | 3/5 | ○ | 4 | 4 | 3.2 | 3.0 |
| | Hexaglycerol dicaprate | 2 | C10 | 6 | 10.7 | | | | | | | | | |
| Comp. Ex. 8 | Hexaglycerol caprylate | 3 | C8 | 6 | 14.5 | 13.0 | 6 | 89 | 6/5 | × | 1 | 3 | not assess-able | not assess-able |
| | Hexaglycerol dicaprate | 2 | C10 | 6 | 10.7 | | | | | | | | | |
| Ex. 17 | Hexaglycerol caprylate | 4.5 | C8 | 6 | 14.5 | 13.0 | 0.5 | 92 | 1/15 | ○ | 3 | 3 | 2.6 | 2.8 |
| | Hexaglycerol dicaprate | 3 | C10 | 6 | 10.7 | | | | | | | | | |
| Ex. 18 | Hexaglycerol caprylate | 4.5 | C8 | 6 | 14.5 | 13.0 | 2 | 90.5 | 4/15 | ◎ | 3 | 4 | 2.8 | 3.0 |
| | Hexaglycerol dicaprate | 3 | C10 | 6 | 10.7 | | | | | | | | | |
| Ex. 19 | Hexaglycerol caprylate | 4.5 | C8 | 6 | 14.5 | 13.0 | 4 | 88.5 | 8/15 | ◎ | 3 | 4 | 2.8 | 3.0 |
| | Hexaglycerol dicaprate | 3 | C10 | 6 | 10.7 | | | | | | | | | |
| Comp. Ex. 9 | Hexaglycerol caprylate | 4.5 | C8 | 6 | 14.5 | 13.0 | 8 | 84.5 | 16/15 | × | 1 | 3 | not assess-able | not assess-able |
| | Hexaglycerol dicaprate | 3 | C10 | 6 | 10.7 | | | | | | | | | |

EP 3 892 256 B1

13

[Table 3]

[Table 3]

Table 3

| | Compound Name | Blending Amount of Components A, B, % by mass | Kind of Fatty Acid | Average Degree of Polymerization of Polyglycerol | HLB | Mixed HLB | Kind of Oil | Blending Amount of Oil, % by mass | Blending Amount of Water, % by mass | C/(A+B) | State of External Appearance | Compatibility with Makeups | Cleansing Power | Feel When Drying | Feel After Drying |
|---|---|---|---|---|---|---|---|---|---|---|---|---|---|---|---|
| Ex. 20 | Hexaglycerol caprylate | 3 | C8 | 6 | 14.5 | 13.0 | Hydrogenated polyisobutene | 1 | 94 | 1/5 | ◎ | 4 | 4 | 2.6 | 3.0 |
| | Hexaglycerol dicaprate | 2 | C10 | 6 | 10.7 | | | | | | | | | | |
| Ex. 21 | Hexaglycerol caprylate | 3 | C8 | 6 | 14.5 | 13.0 | Liquid Paraffin | 1 | 94 | 1/5 | ◎ | 4 | 4 | 3.2 | 2.8 |
| | Hexaglycerol dicaprate | 2 | C10 | 6 | 10.7 | | | | | | | | | | |
| Ex. 3 | Hexaglycerol caprylate | 3 | C8 | 6 | 14.5 | 13.0 | Isononyl isononoate | 1 | 94 | 1/5 | ◎ | 4 | 4 | 3.6 | 3.4 |
| | Hexaglycerol dicaprate | 2 | C10 | 6 | 10.7 | | | | | | | | | | |
| Ex. 22 | Hexaglycerol caprylate | 3 | C8 | 6 | 14.5 | 13.0 | Triethyl hexanoin | 1 | 94 | 1/5 | ◎ | 4 | 3 | 3.0 | 2.8 |
| | Hexaglycerol dicaprate | 2 | C10 | 6 | 10.7 | | | | | | | | | | |
| Ex. 23 | Hexaglycerol caprylate | 3 | C8 | 6 | 14.5 | 13.0 | Olive oil | 1 | 94 | 1/5 | ◎ | 4 | 3 | 2.8 | 2.6 |
| | Hexaglycerol dicaprate | 2 | C10 | 6 | 10.7 | | | | | | | | | | |
| Ex. 24 | Hexaglycerol caprylate | 3 | C8 | 6 | 14.5 | 13.0 | Cyclic cyclohexane | 1 | 94 | 1/5 | ◎ | 4 | 3 | 2.8 | 2.6 |
| | Hexaglycerol dicaprate | 2 | C10 | 6 | 10.7 | | | | | | | | | | |

[Table 4]

[Table 4]

Table 4

| | Compound Name | Blending Amount of Components A, B, % by mass | Kind of Fatty Acid | Average Degree of Polymerization of Polyglycerol | HLB | Mixed HLB | Hydrophilic Surfactant | Blending Amount of Hydrophilic Surfactant, % by mass | Blending Amount of Oil, % by mass | Blending Amount of Water, % by mass | C/(A+B) | State of External Appearance | Compatibility with Makeups | Cleansing Power | High-Temp. Stability, °C | Feel When Drying | Feel After Drying |
|---|---|---|---|---|---|---|---|---|---|---|---|---|---|---|---|---|---|
| Ex 3 | Hexaglycerol caprylate | 3 | C8 | 6 | 14.5 | 13.0 | - | 0 | I | 93.7 | 1/5 | ◎ | 4 | 4 | 45 | 3.6 | 3.4 |
| | Hexaglycerol dicaprate | 2 | C10 | 6 | 10.7 | | | | | | | | | | | | |
| Ex. 25 | Hexaglycerol caprylate | 3 | C8 | 6 | 14.5 | 13.0 | Polyglycerol 4-lauryl ether | 0.3 | 1 | 93.7 | 115 | ◎ | 4 | 4 | 72 | 3.4 | 3.4 |
| | Hexaglycerol dicaprate | 2 | C10 | 6 | 10.7 | | | | | | | | | | | | |
| Ex. 26 | Hexaglycerol caprylate | 3 | C8 | 6 | 14.5 | 13.0 | Polysolvate 20 | 0.3 | 1 | 93.7 | 1/5 | ◎ | 4 | 3 | 61 | 3.2 | 3.4 |
| | Hexaglycerol dicaprate | 2 | C10 | 6 | 10.7 | | | | | | | | | | | | |
| Ex. 27 | Hexaglycerol caprylate | 3 | C8 | 6 | 14.5 | 13.0 | Lauless-15 | 0.3 | 1 | 93.7 | 115 | ◎ | 4 | 4 | 57 | 3.2 | 3.2 |
| | Hexaglycerol dicaprate | 2 | C10 | 6 | 10.7 | | | | | | | | | | | | |

| | Compound Name | Blending Amount of Components A, B, % by mass | Kind of Fatty Acid | Average Degree of Polymerization of Polyglycerol | HLB | Mixed HLB | Hydrophilic Surfactant | Blending Amount of Hydrophilic Surfactant, % by mass | Blending Amount of Oil, % by mass | Blending Amount of Water, % by mass | C/(A+B) | State of External Appearance | Compatibility with Makeups | Cleansing Power | High-Temp. Stability, °C | Feel When Drying | Feel After Drying |
|---|---|---|---|---|---|---|---|---|---|---|---|---|---|---|---|---|---|
| Ex 28 | Hexaglycerol caprylate | 3 | C8 | 6 | 14.5 | 13.0 | Lauryl glucoside | 0.3 | 1 | 93.7 | 115 | ◎ | 4 | 3 | 48 | 3.4 | 3.0 |
| | Hexaglycerol dicaprate | 2 | C10 | 6 | 10.7 | | | | | | | | | | | | |
| Ex. 29 | Hexaglycerol caprylate | 3 | C8 | 6 | 14.5 | 13.0 | Decaglycerol laurate | 0.3 | 1 | 93.7 | 1/5 | ◎ | 4 | 3 | 62 | 3.4 | 3.6 |
| | Hexaglycerol dicaprate | 2 | C10 | 6 | 10.7 | | | | | | | | | | | | |
| Ex. 30 | Hexaglycerol caprylate | 4.5 | C8 | 6 | 14.5 | 13.0 | Polyglycerol 4-lauryl ether | 0.5 | 1 | 92 | 2/15 | ◎ | 4 | 3 | 72 | 3.2 | 3.2 |
| | Hexaglycerol dicaprate | 3 | C10 | 6 | 10.7 | | | | | | | | | | | | |

EP 3 892 256 B1

As is seen from Tables 1 to 4, each of the cosmetic compositions of Examples was all transparent, and had excellent cleansing power and compatibility with makeups, and low stickiness. On the other hand, in cases of Comparative Examples 2 and 4 where Components (A) and (B) were not used in combination, or in cases of Comparative Examples 1, 3, and 5 where even when Components (A) and (B) were used in combination, the mixed HLB was outside the range of from 11.5 to 14.0, the transparency of the external appearance and the cleansing power or the compatibility with makeups would not be satisfactory. In addition, from Table 1, the lowering of skin irritations could be confirmed by using Components (A) and (B) in a combination. In addition, even when Components (A) and (B) were used in a combination and a mixed HLB was within a given range, in Comparative Examples 6 and 7 where the mass ratio of Component (C) to a total amount of Components (A) and (B), i.e. ((C) / (A) + (B)), was smaller than 1/15 to 1, satisfactory cleansing power and compatibility with makeups could not be obtained, and stickiness was also large. In Comparative Examples 8 and 9, the amount of the oil agent blended was large, so that turbidity was caused. In addition, from Table 4, the cosmetic compositions of Examples 26 to 30 further containing a hydrophilic surfactant had excellent high-temperature stability.

(Formulation Examples)

Formulation Examples of the cosmetics of the present invention will be listed hereinbelow, without intending to limit the present invention by these formulation examples. Here, all the blending amounts are expressed in % by mass based on the entire amount of the manufactured articles. The present formulation examples can be prepared by blending various components usually used in the cosmetics to a cosmetic composition (Components 1 to 4 and water in Table 5, or Components 1 to 5 and water in Table 6), in accordance with the applications and purposes, dissolving the entire components at 70°C, and then cooling the solution to room temperature.

[Table 5]

Table 5

| Formulation Example 1: Cleansing Lotion | | |
| --- | --- | --- |
| 1 | Hexaglycerol caprylate | 2.1 |
| 2 | Hexaglycerol dicaprate | 0.9 |
| 3 | Polyglycerol-4 lauryl ether | 0.2 |
| 4 | Isononyl isononanoate | 1 |
| 5 | Preservative | proper amount |
| 6 | pH Adjusting agent | proper amount |
| 7 | Water | up to 100 |

[Table 6]

Table 6

| Form lation Example 2: High-Moisturizing Clean sing Lotion | | |
| --- | --- | --- |
| 1 | Hexaglycerol caprylate | 2.1 |
| 2 | Hexaglycerol dicaprate | 0.9 |
| 3 | Glycerol | 5.0 |
| 4 | BG | 3.0 |
| 5 | Isononyl isononanoate | 1.5 |
| 6 | Preservative | proper amount |
| 7 | pH Adjusting agent | proper amount |

(continued)

| Form lation Example 2: High-Moisturizing Clean sing Lotion | | |
|---|---|---|
| 8 | Water | Up to 100 |

Preparation of Cosmetic Composition

Example 31

Components in a composition as listed in Table 7 were heated to dissolve at 70°C, and the solution was then cooled to room temperature while stirring, to produce 100 g of a cosmetic composition. Here, the blending amount is expressed as % by mass based on the entire amount of the cosmetic composition. The cosmetic composition obtained was freeze-fractured, and subjected to Pt-C vapor deposition to prepare a replica film, and the structure thereof was observed with a transmission electron microscope (manufactured by Hitachi High-Technologies). The observation results are shown in FIG. 1. As is seen in FIG. 1, in the cosmetic composition, oils are absorbed in Components (A) and (B) in large amounts, thereby forming domains of bicontinuous structures. In the cosmetic composition in which a surfactant and an oil agent are at very low concentrations as in the inventive product, examples showing the bicontinuous structures as described above have not so far been confirmed. In addition, the bicontinuous structures support the results that the composition was stained with an oily pigment in the assessment of the compatibility with makeups. Therefore, it is considered to serve as a channel for homogeneously dispersing the oil agent in a bulk, and contribute to improve in the compatibility with makeups. Here, although specific assessment results are not shown for the cosmetic composition of Example 31, the cosmetic composition had excellent cleansing power and compatibility with makeups similar to those in Examples 1 to 30, and had low skin irritability and stickiness.

[Table 7]

Table 7

| Example 31 | | |
|---|---|---|
| 1 | Hexaglycerol caprylate | 2.1 |
| 2 | Hexaglycerol dicaprate | 0.9 |
| 3 | Isononyl isononanoate | 1.5 |
| 4 | Water | 95.5 |
| *Mixed HLB of 1 and 2:13.4 | | |

INDUSTRIAL APPLICABILITY

The cosmetic composition of the present invention has excellent cleansing power and low stickiness, so that the cosmetic composition can be suitably used as aqueous cleansing cosmetics.

**Claims**

1. A cosmetic composition comprising the following components (A) to (D):

Component (A): a polyglycerol fatty acid ester made from a fatty acid having 8 carbon atoms and a polyglycerol having a degree of polymerization of glycerol of from 3 to 14;
Component (B): a polyglycerol fatty acid ester made from a fatty acid having from 10 to 12 carbon atoms and a polyglycerol having a degree of polymerization of glycerol of from 3 to 14;
Component (C): a liquid oil; and
Component (D): water,
wherein a total amount of Component (A) and Component (B) is from 1 to 7.5% by mass, and wherein the mass ratio of Component (C) to a total amount of Components (A) and (B) ((C)/(A)+(B)) is from 1/15 to 1, and wherein Component (A) and Component (B) have a mixed HLB of from 11.5 to 14.0, and wherein the content of said Component (D) is 88.5% by mass or more.

2. The cosmetic composition according to claim 1, further comprising a hydrophilic nonionic surfactant in an amount of 0.01 to 1.0% by mass as Component (E).

3. The cosmetic composition according to claim 2, wherein said Component (E) is a polyglycerol alkyl ether.

4. The cosmetic composition according to any one of claims 1 to 3, which is transparent.

5. Cosmetics comprising a cosmetic composition as defined in any one of claims 1 to 4.

6. The cosmetics according to claim 5, which are cleansing cosmetics.

**Patentansprüche**

1. Eine kosmetische Zusammensetzung, umfassend die folgenden Komponenten (A) bis (D):

   Komponente (A): ein Polyglycerinfettsäureester, hergestellt aus einer Fettsäure mit 8 Kohlenstoffatomen und einem Polyglycerin mit einem Glycerin-Polymerisationsgrad von 3 bis 14;
   Komponente (B): ein Polyglycerinfettsäureester, hergestellt aus einer Fettsäure mit 10 bis 12 Kohlenstoffatomen und einem Polyglycerin mit einem Glycerin-Polymerisationsgrad von 3 bis 14;
   Komponente (C): ein flüssiges Öl; und
   Komponente (D): Wasser,
   wobei eine Gesamtmenge an Komponente (A) und Komponente (B) von 1 bis 7,5 Massen-% beträgt und wobei das Massenverhältnis von Komponente (C) zu einer Gesamtmenge an Komponenten (A) und (B) ((C)/(A)+(B)) von 1/15 bis 1 beträgt und wobei Komponente (A) und Komponente (B) einen gemischten HLB-Wert von 11,5 bis 14,0 aufweisen und wobei der Gehalt der Komponente (D) 88,5 Massen-% oder mehr beträgt.

2. Die kosmetische Zusammensetzung nach Anspruch 1, weiter umfassend ein hydrophiles nicht-ionisches grenzflächenaktives Mittel in einer Menge von 0,01 bis 1,0 Massen-% als Komponente (E).

3. Die kosmetische Zusammensetzung nach Anspruch 2, wobei die Komponente (E) ein Polyglycerinalkylether ist.

4. Die kosmetische Zusammensetzung nach einem der Ansprüche 1 bis 3, welche transparent ist.

5. Kosmetik, umfassend eine wie in einem der Ansprüche 1 bis 4 definierte kosmetische Zusammensetzung.

6. Die Kosmetik nach Anspruch 5, bei welcher es sich um Reinigungskosmetik handelt.

**Revendications**

1. Composition cosmétique comprenant les composants (A) à (D) suivants :

   composant (A) : un ester d'acide gras et de polyglycérol fait à partir d'un acide gras ayant 8 atomes de carbone et d'un polyglycérol ayant un degré de polymérisation de glycérol de 3 à 14 ;
   composant (B) : un ester d'acide gras et de polyglycérol fait à partir d'un acide gras ayant 10 à 12 atomes de carbone et d'un polyglycérol ayant un degré de polymérisation de glycérol de 3 à 14 ;
   composant (C) : une huile liquide ; et
   composant (D) : de l'eau,
   dans laquelle la quantité totale de composant (A) et de composant (B) est de 1 à 7,5 % en masse, et dans laquelle le rapport en masse du composant (C) à la quantité totale des composants (A) et (B) ((C)/(A)+(B)) est de 1/15 à 1, et dans laquelle le composant (A) et le composant (B) ont un indice HLB mélangé de 11,5 à 14,0, et dans laquelle la teneur en ledit composant (D) est de 88,5 % en masse ou plus.

2. Composition cosmétique selon la revendication 1, comprenant en outre un tensioactif non-ionique hydrophile en une quantité de 0,01 à 1,0 % en masse en tant que composant (E).

3. Composition cosmétique selon la revendication 2, dans laquelle ledit composant (E) est un éther alkylique de polyglycérol.

4. Composition cosmétique selon l'une quelconque des revendications 1 à 3, qui est transparente.

5. Cosmétiques comprenant une composition cosmétique telle que définie dans l'une quelconque des revendications 1 à 4.

6. Cosmétiques selon la revendication 5, qui sont des cosmétiques nettoyants.

[FIG. 1]

**EP 3 892 256 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014210744 A